# EUROPEAN PATENT SPECIFICATION

(11) **EP 4 289 472 B1**
(45) Date of publication and mention of the grant of the patent: **23.07.2025**
(21) Application number: 21924878.8
(22) Date of filing: 24.12.2021
(51) Int. Cl.: A61N 5/06

(54) **LIGHT IRRADIATION HAIR REMOVAL DEVICE**
LICHTBESTRAHLUNGSVORRICHTUNG ZUR HAARENTFERNUNG
DISPOSITIF D'ÉPILATION PAR IRRADIATION DE LUMIÈRE

(30) Priority: 05.02.2021 JP 2021017231
(43) Date of publication of application: 13.12.2023
(73) Proprietor: Panasonic Intellectual Property Management Co., Ltd., Kadoma-shi, Osaka 571-0057 (JP)
(72) Inventor: SAKAMOTO, Ryohei, Kadoma-shi, Osaka 571-0057 (JP); KASUGAI, Hideki, Kadoma-shi, Osaka 571-0057 (JP)
(74) Representative: Grünecker Patent- und Rechtsanwälte PartG mbB
(86) International application number: PCT/JP2021/048150
(87) International publication number: WO 2022/168493

(56) References cited:
- WO-A1-2011/037122
- WO-A2-2005/074830
- JP-A- 2009 240 690
- JP-A- 2013 111 391
- JP-A- 2021 010 741
- US-A1- 2013 184 693
- US-A1- 2015 025 601

## Description

### TECHNICAL FIELD

The present disclosure relates to a light irradiation hair removal device.

### BACKGROUND ART

Conventionally, a light irradiation hair removal device that emits light to remove hair is known. The light irradiation hair removal device promotes a discharge of hair by irradiating a skin surface of a user with a light having a specific wavelength and causing the light to act on melanin of hair follicles. As a light irradiation hair removal device, for example, a device as shown in PTL 1 is known.

PTL 1 discloses a light irradiation hair removal device having a light source that causes a processing light and a sensing light to be incident on a target object, a light detector that detects the sensing light for sensing the target object, and a control unit for controlling the light source. The control unit determines absorption of the sensing light from the detected sensing light, and controls the light source such that the processing light is generated depending on the determined absorption.

### Citation List

### Patent Literature

PTL 1: Japanese Patent No. 5715128 A further relevant prior art is formed by document US 2013/0184693 A1.

### SUMMARY OF THE INVENTION

A conventional light irradiation hair removal device includes a vertical cavity surface emitting laser (VCSEL) with mutually-independent sub-groups that emit light of different wavelengths. Then, the conventional light irradiation hair removal device controls light turning on and off of these VCSEL in the generation of the processing light dependent on the absorption of the sensing light. In the conventional light irradiation hair removal device, since a part of the mounted VCSEL is turned off to generate the processing light, an irradiance may be lowered and a hair removal effect may be reduced. Further, in the conventional light irradiation hair removal device, in a case where a light having a single wavelength is emitted, a light depth entering the skin becomes constant, and melanocytes containing melanin of the hair follicles existing at a specific depth may be intensively heated. In such a case, uneven heating in a depth direction of the skin occurs, and thus a sufficient hair removal effect may not be obtained.

The invention is defined in the appended set of claims. The present disclosure provides a light irradiation hair removal device capable of uniformly irradiating the entire area of melanocytes distributed in hair follicles with light.

A light irradiation hair removal device according to one aspect of the present disclosure includes a first light source, a second light source, a skin cooling unit, a push switch, a first cooling unit, and a second cooling unit. The first light source emits first light having a wavelength from 400 nm to 1200 nm inclusive. The second light source emits second light having a wavelength from 400 nm to 1200 nm inclusive. The skin cooling unit faces the first light source and the second light source, transmits the first light emitted from the first light source and the second light emitted from the second light source, and cools a skin when the skin cooling unit comes into contact with the skin. The push switch includes a pressing unit surrounding a periphery of the first light source, the second light source, and the skin cooling unit. In a case where the pressing unit is not pressed, the pressing unit protrudes toward a direction opposite to the first light source and the second light source against the skin cooling unit from a surface of the skin cooling unit in contact with the skin. In a case where the pressing unit is pressed by the skin, a surface pressed by the skin moves toward the direction of the first light source and the second light source against the skin cooling unit. The push switch switches between emission and non-emission of the first light from the first light source and the second light from the second light source. The first light is emitted from the first light source and the second light is emitted from the second light source during at least a part of time while the pressing unit is pressed, and the first light is not emitted from the first light source and the second light is not emitted from the second light source while the pressing unit is not pressed. The first cooling unit cools the first light source. The second cooling unit cools the second light source. By cooling the first light source, the first cooling unit shifts the wavelength of the first light emitted from the first light source to a wavelength different from the wavelength of the second light emitted from the second light source.

According to the present disclosure, it is possible to obtain a light irradiation hair removal device capable of uniformly irradiating the entire area of melanocytes distributed in hair follicles with light.

### BRIEF DESCRIPTION OF THE DRAWINGS

Fig. 1 is a cross-sectional view illustrating a configuration of a light irradiation hair removal device according to the present exemplary embodiment.
Fig. 2 is a cross-sectional view taken along line II-II in Fig. 1.
Fig. 3 is a perspective view illustrating an example of an arrangement state of a first light source and a second light source according to the present exemplary embodiment.
Fig. 4 is a control block diagram according to a controller.
Fig. 5 is a cross-sectional view illustrating an example of a state before use of the light irradiation hair removal device.
Fig. 6 is a cross-sectional view illustrating an example of a state of the light irradiation hair removal device before a push switch is pressed.
Fig. 7 is a cross-sectional view illustrating an example of a state of the light irradiation hair removal device after the push switch is pressed.
Fig. 8 is a cross-sectional view illustrating an example of a state of the light irradiation hair removal device while the skin is irradiated with light.

### DESCRIPTION OF EMBODIMENT

Hereinafter, exemplary embodiments will be described in detail with reference to the drawings. However, detailed descriptions more than necessary may be omitted. For example, detailed descriptions of already well-known matters or redundant descriptions of substantially the same configuration may be omitted.

Note that, the accompanying drawings and the following description are only presented to help those skilled in the art fully understand the present disclosure, and are not intended to limit the subject matters described in the scope of claims.

Further, in the following exemplary embodiment, up-down direction Z of light irradiation hair removal device 1 is defined with an emission port as an upward direction and a direction opposite to the emission port as a downward direction. Further, a direction in a horizontal direction of light irradiation hair removal device 1 is defined as width direction Y, and a direction orthogonal to up-down direction Z and width direction Y is defined as front-back direction X.

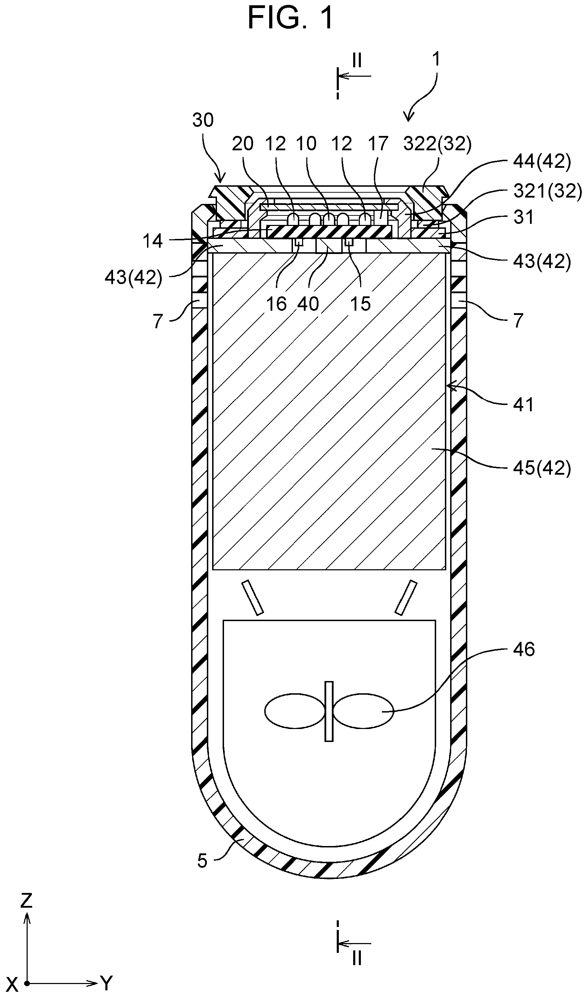

Hereinafter, light irradiation hair removal device 1 according to the present exemplary embodiment will be described with reference to Figs. 1 to 8.

### [Configuration]

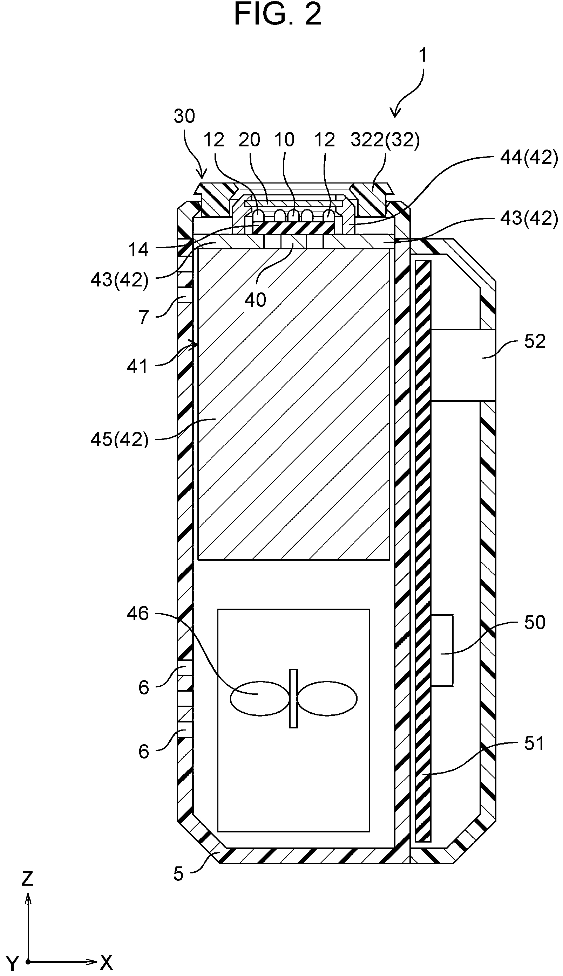

Fig. 1 is a cross-sectional view illustrating the configuration of light irradiation hair removal device 1 according to the present exemplary embodiment, and Fig. 2 is a cross-sectional view taken along line II-II in Fig. 1. As shown in Figs. 1 and 2, light irradiation hair removal device 1 includes housing 5, first light source 10, second light source 12, first temperature sensor 15, second temperature sensor 16, third temperature sensor 17, skin cooling unit 20, and push switch 30. Furthermore, light irradiation hair removal device 1 includes first cooling unit 40, second cooling unit 41, and controller 50.

One end of housing 5 is provided with an opening portion serving as a light emission port of light irradiation hair removal device 1. First light source 10 and second light source 12 are provided in the opening portion of housing 5, and light is emitted on skin S of a person. Further, a bottom portion is formed on a side of housing 5 opposite to first light source 10 and second light source 12. Housing 5 is provided with a plurality of first opening portions 6 and a plurality of second opening portions 7, and external air is taken in from the plurality of first opening portions 6 and discharged from the plurality of second opening portions 7. Inside housing 5, first light source 10, second light source 12, first temperature sensor 15, second temperature sensor 16, third temperature sensor 17, skin cooling unit 20, push switch 30, first cooling unit 40, second cooling unit 41, and controller 50 are accommodated.

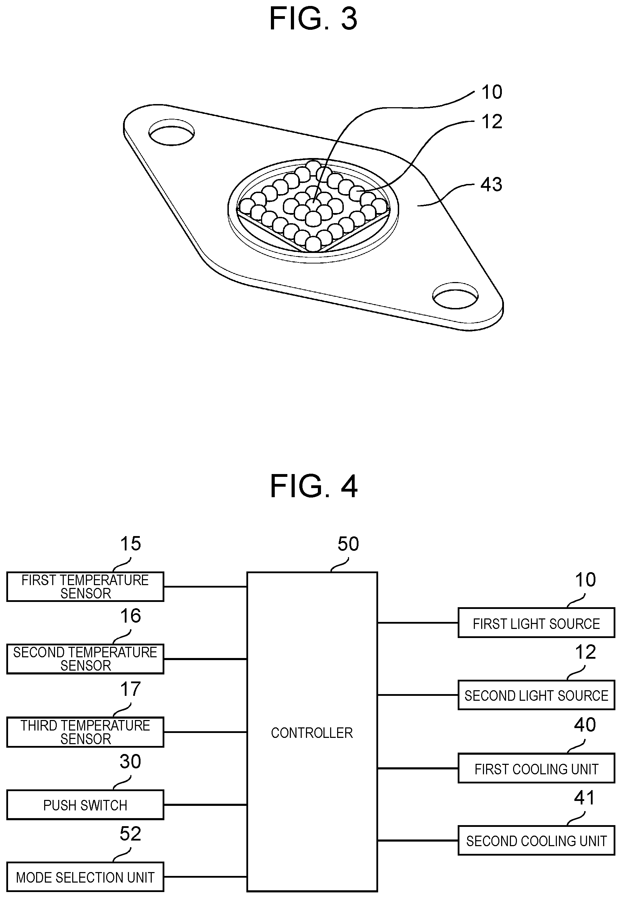

Fig. 3 is a perspective view illustrating an example of a schematic arrangement state of first light source 10 and second light source 12 according to the present exemplary embodiment. Note that, in Fig. 3, configurations of third temperature sensor 17, skin cooling unit 20, push switch 30, and second cooling unit 41 are partially omitted. As shown in Figs. 1 to 3, first light source 10 is disposed substantially at a center of substrate 14, and second light source 12 is disposed so as to surround first light source 10. In the present exemplary embodiment, first light source 10 includes a plurality of light emitting diodes (LED). Further, second light source 12 includes a plurality of LEDs. The plurality of LEDs included in first light source 10 are mounted on substrate 14 in a state of being spaced at substantially equal intervals. Further, the plurality of LEDs included in second light source 12 are mounted on substrate 14 in a state of being spaced at substantially equal intervals. First light source 10 and second light source 12 are disposed with a gap larger than a gap between each LED included in first light source 10. First light source 10 and second light source 12 are electrically connected to a power source (not illustrated), and when power is supplied from the power source, light is emitted from first light source 10 and second light source 12.

First light source 10 and second light source 12 emit light having a wavelength from 400 nm to 1200 nm inclusive. When the light as described above is emitted on skin S, melanin of hair follicles absorbs the light and generates heat. Then, hair matrixes included in the hair follicles are damaged by the heat, and a hair discharge is promoted. A wavelength of light may be more than or equal to 500 nm, more than or equal to 600 nm, more than or equal to 700 nm, or more than or equal to 800 nm. Further, a wavelength of light may be less than or equal to 1000 nm, or less than or equal to 900 nm. The light emitted from first light source 10 and second light source 12 may be light having a peak wavelength within a range from 400 nm to 1200 nm inclusive. Even in a case where light has a peak wavelength within the range as described above, the emitted light may contain a wavelength component outside the above range. Note that, the wavelength is a wavelength of light emitted in a case where the temperature of first light source 10 or second light source 12 is 25°C. Further, types of first light source 10 and second light source 12 may be the same, and the wavelength of light emitted from first light source 10 and the wavelength of light emitted from second light source 12 may be the same at a predetermined temperature. Further, first light source 10 and second light source 12 may use a combination of LEDs that emit light of different wavelengths.

First light source 10 and second light source 12 preferably emit light under a condition that an irradiance is from 15 W/cm² to 50 W/cm² inclusive. By irradiating the hair with light at an irradiance more than or equal to 15 W/cm², it is possible to achieve a good hair removal effect on hair from an early growth period to a growth period. Further, by irradiating the skin with light at an irradiance less than or equal to 50 W/cm², it is possible to suppress a rise in skin temperature due to light irradiation. Therefore, skin S is cooled more reliably, and skin irritation can be reduced. An irradiance may be more than or equal to 20 W/cm², more than or equal to 25 W/cm², or more than or equal to 30 W/cm². Further, an irradiance may be less than or equal to 45 W/cm², or less than or equal to 40 W/cm².

In the present exemplary embodiment, the light emitted from first light source 10 and second light source 12 is pulsed light emitted in an intermittent manner. First light source 10 and second light source 12 preferably intermittently emit light under a condition that irradiation time is from 500 ms to 1000 ms inclusive. By irradiating the hair with light for more than or equal to 500 ms, it is possible to achieve a good hair removal effect on hair from the early growth period to the growth period. Further, by irradiating the skin with light for less than or equal to 1000 ms, it is possible to suppress a rise in skin temperature due to light irradiation. Therefore, skin S is cooled more reliably, and skin irritation can be reduced. The irradiation time may be more than or equal to 600 ms. Further, the irradiation time may be less than or equal to 900 ms, or less than or equal to 800 ms.

Energy of each pulsed light emitted from first light source 10 and second light source 12 is preferably from 9 J/cm² to 50 J/cm² inclusive. When the energy is more than or equal to 9 J/cm², a good hair removal effect can be achieved. Further, in light irradiation hair removal device 1 including skin cooling unit 20, when the energy is less than or equal to 50 J/cm², a rise in skin temperature due to light irradiation can be suppressed. Therefore, skin S is cooled more reliably, and skin irritation can be reduced.

First temperature sensor 15 detects the temperature of first light source 10. First temperature sensor 15 is provided on a surface of substrate 14 opposite to first light source 10 so as to face first light source 10 across substrate 14. Then, first temperature sensor 15 indirectly measures the temperature of first light source 10 by measuring the temperature of substrate 14. In the present exemplary embodiment, first temperature sensor 15 includes a thermistor that is a contact temperature sensor. However, first temperature sensor 15 is not limited to a thermistor, and may include a contact temperature sensor such as a thermocouple or a resistance temperature detector, or a non-contact temperature sensor such as a radiation thermometer. Further, as long as first temperature sensor 15 can detect the temperature of first light source 10, a position provided in first temperature sensor 15 is not particularly limited.

Second temperature sensor 16 detects the temperature of second light source 12. Second temperature sensor 16 is provided on a surface of substrate 14 opposite to second light source 12 so as to face second light source 12 across substrate 14. Then, second temperature sensor 16 indirectly measures the temperature of second light source 12 by measuring the temperature of substrate 14. In the present exemplary embodiment, second temperature sensor 16 includes a thermistor that is a contact temperature sensor. However, second temperature sensor 16 is not limited to a thermistor, and may include a contact temperature sensor such as a thermocouple or a resistance temperature detector, or a non-contact temperature sensor such as a radiation thermometer. Further, as long as second temperature sensor 16 can detect the temperature of second light source 12, a position provided in second temperature sensor 16 is not particularly limited.

Skin cooling unit 20 is disposed at a position facing first light source 10 and second light source 12. Skin cooling unit 20 may be in contact with first light source 10 and second light source 12, or may be disposed with a space from first light source 10 and second light source 12. Further, skin cooling unit 20 is provided so as to be in contact with skin S on a side opposite to first light source 10 and second light source 12. Skin cooling unit 20 is made of a material having translucency. When first light source 10 and second light source 12 emit light, skin cooling unit 20 transmits light emitted from first light source 10 and second light source 12, and skin S is irradiated with light transmitted through skin cooling unit 20. Skin cooling unit 20 is, for example, a plate having translucency, and in the present exemplary embodiment, skin cooling unit 20 of a disk is used.

Skin cooling unit 20 is preferably made of a material that is unlikely to absorb light emitted from first light source 10 and second light source 12. Specifically, a total light transmittance of skin cooling unit 20 is preferably more than or equal to 80%. When the total light transmittance is more than or equal to 80%, most of light emitted from first light source 10 and second light source 12 can be transmitted through skin cooling unit 20. Therefore, a large amount of light can reach melanin, which can promote the hair removal effect. Further, since the amount of light absorbed by skin cooling unit 20 and converted into heat can be reduced, a temperature rise of skin cooling unit 20 can be suppressed. From a viewpoint of making it difficult for skin cooling unit 20 to absorb light, the total light transmittance is more preferably more than or equal to 90%, still more preferably more than or equal to 95%, and particularly preferably more than or equal to 99%. An upper limit value of the total light transmittance is 100%. The total light transmittance can be measured according to JIS K7361-1:1997.

A refractive index of skin cooling unit 20 is preferably more than or equal to 1.7. When the refractive index of skin cooling unit 20 is more than or equal to 1.7, light from first light source 10 and second light source 12 is not easily absorbed by skin cooling unit 20. Skin cooling unit 20 becomes easier to transmit light as the refractive index value increases. Therefore, the refractive index is more preferably more than or equal to 1.8, still more preferably more than or equal to 1.9, and particularly preferably more than or equal to 2.0. The upper limit value of the refractive index is not particularly limited, but may be 10. The refractive index can be measured by minimum deviation method according to JIS B7071-1:2015.

Skin cooling unit 20 cools skin S when it comes into contact with skin S. Skin cooling unit 20 preferably includes a material having a high thermal conductivity. A thermal conductivity of skin cooling unit 20 is preferably more than or equal to 1 W/mK. When the thermal conductivity is more than or equal to 1 W/mK, even when skin cooling unit 20 is heated by light from first light source 10 and second light source 12 and skin S, heat is easily dissipated, and thus skin S can be effectively cooled. As a value of the thermal conductivity increases, the thermal conductivity of skin cooling unit 20 tends to increase, and a cooling effect of skin cooling unit 20 tends to be better. Therefore, from a viewpoint of cooling efficiency, the thermal conductivity of skin cooling unit 20 is more preferably more than or equal to 2 W/mK, still more preferably more than or equal to 10 W/mK, particularly preferably more than or equal to 30 W/mK, and most preferably more than or equal to 100 W/mK. The upper limit value of the thermal conductivity is not particularly limited, but may be 100,000 W/mK. The thermal conductivity can be measured by laser flash method according to JIS R1611:2010.

Skin cooling unit 20 may contain an inorganic substance. Specifically, skin cooling unit 20 preferably includes at least one selected from the group consisting of Al₂O₃, ZnO, ZrO₂, MgO, GaN, AlN, and diamond. Since these materials have a high refractive index and a high thermal conductivity, the translucency and thermal conductivity of skin cooling unit 20 can be improved. Note that, Al₂O₃ (sapphire) has a refractive index of 1.79 and a thermal conductivity of 42 W/mK. ZnO has a refractive index of 2.01 and a thermal conductivity of 20 W/mK. ZrO₂ has a refractive index of 2.13 and a thermal conductivity of 3 W/mK. MgO has a refractive index of 1.74 and a thermal conductivity of 47 W/mK. GaN has a refractive index of 2.346 and a thermal conductivity of 200 W/mK. AlN has a refractive index of 2.175 and a thermal conductivity of 150 W/mK. Diamond has a refractive index of 2.417 and a thermal conductivity of 1000 W/mK.

Skin cooling unit 20 may contain a resin such as a silicone resin from a viewpoint of heat resistance and translucency. Further, skin cooling unit 20 may include a resin such as a silicone resin and a highly thermally conductive filler dispersed in the resin. Since skin cooling unit 20 includes the highly thermally conductive filler, heat of skin cooling unit 20 is easily dissipated, and thus skin S can be effectively cooled. The highly thermally conductive filler may contain an inorganic substance as described above.

A proportion of the inorganic substance in skin cooling unit 20 is preferably more than or equal to 10 mass%. By setting the proportion of the inorganic substance in skin cooling unit 20 to more than or equal to 10 mass%, the thermal conductivity of skin cooling unit 20 can be improved. The proportion of the inorganic substance in skin cooling unit 20 is more preferably more than or equal to 30 mass%, still more preferably more than or equal to 50 mass%, particularly preferably more than or equal to 70 mass%, and most preferably more than or equal to 90 mass%.

Skin cooling unit 20 is preferably cooled to be from -5°C to 35°C inclusive. By cooling skin cooling unit 20 to be more than or equal to -5°C, it is possible to cool skin S so that pain in skin S caused by the cooling is unlikely to occur. On the other hand, when skin cooling unit 20 is cooled to be less than or equal to 35°C, inflammation due to a rise in skin temperature during light irradiation can be suppressed. Skin cooling unit 20 is more preferably cooled to be more than or equal to 0°C, still more preferably more than or equal to 5°C, and particularly preferably more than or equal to 10°C. Further, skin cooling unit 20 is more preferably cooled to be less than or equal to 30°C, still more preferably less than or equal to 25°C, particularly preferably less than or equal to 20°C, and most preferably less than or equal to 15°C.

Third temperature sensor 17 detects the temperature of skin cooling unit 20. By detecting the temperature of skin cooling unit 20, the temperature of skin cooling unit 20 can be precisely controlled. Third temperature sensor 17 is provided so as to face skin cooling unit 20. Specifically, third temperature sensor 17 is provided on substrate 14. In the present exemplary embodiment, third temperature sensor 17 includes a contact temperature sensor. Examples of the contact temperature sensor include a thermistor, a thermocouple, and a resistance temperature detector.

Push switch 30 is a self-reset switch. Push switch 30 is provided at connection unit 43 of second cooling unit 41. Push switch 30 is disposed outside first light source 10, second light source 12, and skin cooling unit 20 in front-back direction X and width direction Y, and is provided so as to surround first light source 10, second light source 12, and skin cooling unit 20. Push switch 30 includes two bases 31 and pressing unit 32.

Two bases 31 are fixed to connection unit 43 outside grip unit 44 of second cooling unit 41 such that first light source 10, second light source 12, and skin cooling unit 20 are disposed therebetween in width direction Y. Bases 31 have a quadrangular prism shape extending upward from connection unit 43.

Pressing unit 32 is engaged with bases 31, and moves in up-down direction Z by being pressed by skin S. Pressing unit 32 surrounds a periphery of first light source 10, second light source 12, and skin cooling unit 20. Pressing unit 32 includes two first components 321 and one second component 322.

First component 321 has a columnar shape extending upward in up-down direction Z from bases 31, and is provided at a substantially central portion of bases 31 in front-back direction X and width direction Y, respectively. Second component 322 is disposed so as to be in contact with a surface of first component 321 opposite to bases 31. Second component 322 has a through hole at a center in front-back direction X and width direction Y, and has a donut shape extending in up-down direction Z. First light source 10, second light source 12, and skin cooling unit 20 are disposed in the through hole of second component 322. A part of a surface of second component 322 protrudes upward in up-down direction Z from a surface of skin cooling unit 20 opposite to first light source 10 and second light source 12. Note that, in the present exemplary embodiment, first component 321 and second component 322 are different components separated from each other, but pressing unit 32 may be formed by one component that is continuously integrally formed. Further, the number of bases 31, first component 321, and second component 322 is not particularly limited, and can be changed as appropriate.

In a case where pressing unit 32 is not pressed, it protrudes toward a direction opposite to first light source 10 and second light source 12 (upward in up-down direction Z) against skin cooling unit 20 from a surface of skin cooling unit 20 in contact with skin S. A contact (not illustrated) is provided inside bases 31 and pressing unit 32. While pressing unit 32 is not pressed, push switch 30 is configured such that a circuit to which first light source 10 and second light source 12 are connected is opened without contact between a contact of bases 31 and a contact of pressing unit 32. On the other hand, in a case where pressing unit 32 is pressed, a surface pressed by skin S moves toward the direction of first light source 10 and second light source 12 (downward in up-down direction Z) against skin cooling unit 20. Therefore, a contact provided in bases 31 and a contact provided in pressing unit 32 come into contact with each other, whereby the circuit to which first light source 10 and second light source 12 are connected is closed.

An elastic body (not illustrated) is provided between bases 31 and pressing unit 32. In a case where pressing unit 32 is pressed, the elastic body is elastically deformed, and pushes back pressing unit 32 by an elastic force generated by an elastic deformation. Therefore, when the force that presses pressing unit 32 is removed, the elastic body acts on pressing unit 32 so as to return pressing unit 32 to its original position, and thus a contact surface of pressing unit 32 with skin S moves toward a direction opposite to bases 31 (upward in up-down direction Z).

Push switch 30 switches between emission and non-emission of light from first light source 10 and second light source 12. Light is emitted from first light source 10 and second light source 12 during at least a part of time while pressing unit 32 is pressed. While pressing unit 32 is not pressed, light is not emitted from first light source 10 and second light source 12. Therefore, it is configured such that skin S is irradiated with light during at least a part of time while light irradiation hair removal device 1 is pressed against skin S, and the irradiation of light is stopped when light irradiation hair removal device 1 is separated from skin S.

Light may be emitted immediately after push switch 30 is pressed, or may be emitted after a predetermined time has elapsed since push switch 30 was pressed. A timing at which light is emitted from first light source 10 and second light source 12 may be controlled by controller 50. Light irradiation hair removal device 1 may emit light from first light source 10 and second light source 12 after skin S comes into contact with a surface of skin cooling unit 20. As a result, the skin surface is irradiated with light in a cooled state. Therefore, since heat generation of skin S is suppressed, irritation to the skin S can be suppressed. Further, since skin S is irradiated with light in contact with skin cooling unit 20, an uneven irradiation can be suppressed, and a stable hair removal effect can be obtained.

First cooling unit 40 cools first light source 10. First cooling unit 40 is provided on a surface of substrate 14 opposite to first light source 10 so as to face first light source 10 across substrate 14. Then, first cooling unit 40 cools first light source 10 via substrate 14. First cooling unit 40 may cool first light source 10 so that the temperature of first light source 10 becomes lower than the temperature of second light source 12. Note that, in the present exemplary embodiment, first cooling unit 40 includes a Peltier element. Then, one surface of first cooling unit 40 is connected to substrate 14, and heat dissipation fin 45 of second cooling unit 41 is connected to the other surface. However, first cooling unit 40 is not particularly limited as long as it can cool first light source 10.

Second cooling unit 41 cools second light source 12. Second cooling unit 41 is provided on a surface of substrate 14 opposite to second light source 12 so as to face second light source 12 across substrate 14. Specifically, second cooling unit 41 is connected to an edge of substrate 14. Then, second cooling unit 41 cools second light source 12 via substrate 14. In the present exemplary embodiment, second cooling unit 41 includes an air-cooled cooler.

Further, second cooling unit 41 cools skin cooling unit 20. Since light irradiation hair removal device 1 includes second cooling unit 41, the temperature of skin cooling unit 20 can be further lowered. Therefore, a skin cooling effect by skin cooling unit 20 can be further improved. Second cooling unit 41 includes heat dissipation unit 42 and air blower 46.

Heat dissipation unit 42 is connected to skin cooling unit 20, and dissipates heat taken from skin cooling unit 20. Heat dissipation unit 42 includes connection unit 43, grip unit 44, and heat dissipation fin 45.

Connection unit 43 is a plate-shaped member having an opening at the central portion. Substrate 14 is provided on a first surface, which is one surface of connection unit 43. Substrate 14 is smaller than connection unit 43, and is provided so as to be accommodated inside connection unit 43. Grip unit 44 and push switch 30 are connected to outside of substrate 14 on the first surface of connection unit 43. Heat dissipation fin 45 is provided on a second surface, which is a surface opposite to the first surface of connection unit 43. First cooling unit 40, first temperature sensor 15, and second temperature sensor 16 are disposed in the opening of connection unit 43.

Grip unit 44 protrudes upward in up-down direction Z from the first surface of connection unit 43, and grips the entire peripheral edge of skin cooling unit 20. Therefore, first light source 10 and second light source 12 are surrounded by skin cooling unit 20, grip unit 44, and connection unit 43. The heat generated by first light source 10 and second light source 12 is dissipated via skin cooling unit 20 and heat dissipation unit 42 of second cooling unit 41. Note that, while grip unit 44 grips the entire peripheral edge of skin cooling unit 20, grip unit 44 may be connected to at least a part of skin cooling unit 20.

Heat dissipation fin 45 is provided on the second surface of connection unit 43, which is a surface opposite to first light source 10 and second light source 12. Therefore, the heat of skin cooling unit 20 and first cooling unit 40 moves to heat dissipation fin 45 through grip unit 44 and connection unit 43. Heat dissipation fin 45 includes a plurality of fins, and has a large contact area with air, so that heat is easily dissipated.

Heat dissipation unit 42 preferably contains a material excellent in thermal conductivity. The value of the thermal conductivity of heat dissipation unit 42 may be larger than the value of the thermal conductivity of skin cooling unit 20. Specifically, heat dissipation unit 42 may contain a metal such as aluminum, iron, or copper. Grip unit 44, connection unit 43, and heat dissipation fin 45 may be made of the same material, or may be made of different materials.

Air blower 46 cools heat dissipation unit 42 by sending air to heat dissipation unit 42. Air blower 46 includes, for example, a fan, and when the fan rotates, an air flow is generated. Housing 5 is provided with a plurality of first opening portions 6 at positions facing air blower 46. Further, housing 5 is provided with a plurality of second opening portions 7 at positions facing heat dissipation fin 45. Therefore, when air blower 46 is driven, air taken in from outside of housing 5 through the plurality of first opening portions 6 is sent to heat dissipation fin 45. The heat of the air in contact with heat dissipation fin 45 is exchanged with the heat of heat dissipation fin 45, and heat dissipation fin 45 is cooled. The air heated in contact with heat dissipation fin 45 is discharged to outside of housing 5 through the plurality of second opening portions 7.

Fig. 4 is a control block diagram according to controller 50. Controller 50 is provided on substrate 51 (see Fig. 2). Controller 50 controls emission and non-emission of light from first light source 10 and second light source 12. Further, controller 50 controls a drive and stop of first cooling unit 40 and second cooling unit 41. As shown in Fig. 4, first temperature sensor 15, second temperature sensor 16, third temperature sensor 17, push switch 30, and mode selection unit 52 are connected to an input side of controller 50. On the other hand, first light source 10, second light source 12, first cooling unit 40, and second cooling unit 41 are connected to an output side of controller 50. Controller 50 has a computer system including a central processing unit (CPU), a read only memory (ROM), and a random access memory (RAM). Then, when the CPU executes programs stored in the ROM, the computer system functions as controller 50. Here, the programs executed by the CPU are recorded in advance in the ROM of the computer system, but may be provided by being recorded in a non-transitory recording medium such as a memory card, or may be provided through a telecommunication line such as the Internet.

Controller 50 turns on or blinks first light source 10 and second light source 12 in a case where push switch 30 is pressed. Controller 50 may cause first light source 10 and second light source 12 to emit light simultaneously when push switch 30 is pressed, or may cause first light source 10 and second light source 12 to emit light after a predetermined time has elapsed since push switch 30 was pressed.

Controller 50 may cause second cooling unit 41 to cool skin cooling unit 20 so that the temperature of skin cooling unit 20 is from -5°C to 35°C inclusive. Controller 50 may receive signals related to the temperature of skin cooling unit 20 from third temperature sensor 17, and drive second cooling unit 41 so as to cool skin cooling unit 20 based on the signals. Controller 50 may cool skin cooling unit 20 by controlling an output of air blower 46 or the like.

Controller 50 may control the cooling by first cooling unit 40 so as to cool first light source 10 to a temperature different from that of second light source 12 based on the temperature of first light source 10 detected by first temperature sensor 15 and the temperature of second light source 12 detected by second temperature sensor 16. Then, controller 50 may shift the wavelength of light emitted from first light source 10 to a wavelength different from the wavelength of light emitted from second light source 12 by controlling the cooling by first cooling unit 40.

Specifically, controller 50 acquires signals related to the temperature of first light source 10 detected by first temperature sensor 15. Further, controller 50 acquires signals related to the temperature of second light source 12 detected by second temperature sensor 16. In a case where the temperature of first light source 10 is different from the temperature of second light source 12, controller 50 does not cause first cooling unit 40 to cool first light source 10, but acquires again the signals related to the temperatures of first light source 10 and second light source 12 from first temperature sensor 15 and second temperature sensor 16 and compares them. On the other hand, in a case where the temperature of first light source 10 and the temperature of second light source 12 are the same, controller 50 causes first cooling unit 40 to cool first light source 10.

Controller 50 may cause first cooling unit 40 to cool first light source 10 so that the temperature of first light source 10 becomes lower than the temperature of second light source 12. For example, controller 50 compares the temperature of first light source 10 with the temperature of second light source 12, and causes first cooling unit 40 to cool first light source 10 in a case where the temperature of first light source 10 is more than or equal to the temperature of second light source 12. Further, in a case where the temperature of first light source 10 is less than or equal to a threshold value, controller 50 stops cooling first light source 10.

Controller 50 may cause first cooling unit 40 to control the temperature of first light source 10 so as to be in a predetermined temperature range according to parts of skin S irradiated with light from first light source 10 and light from second light source 12. Hair follicles vary in size depending on each part. For example, hair follicles of beards are large and hair follicles of downy hair are small. Therefore, by controlling the temperature of first light source 10 according to each part, each part can be irradiated with light having an optimum wavelength. Controller 50 may read upper limit and lower limit threshold temperature values stored in a storage (not illustrated), and cause first cooling unit 40 to cool first light source 10. Then, controller 50 may cause first cooling unit 40 to cool first light source 10 so as to be within a range from the upper limit threshold temperature value to the lower limit threshold temperature value corresponding to each predetermined part. The part to be irradiated with light may be set by a user via mode selection unit 52, or may be determined by controller 50 according to an outer appearance of hair.

Mode selection unit 52 is connected to substrate 51, and at least a part thereof is exposed on the outer surface. Mode selection unit 52 is provided with a switch (not illustrated) capable of selecting a part of skin S on the exposed outer surface. Mode selection unit 52 may include, for example, one switch capable of switching each part every time it is pressed. Further, mode selection unit 52 may include a plurality of switches corresponding to the respective parts, and the respective parts selected by pressing the respective switches may be selected. The switch may be a push button switch or a touch panel switch.

### [Operation]

The following describes operations and actions of light irradiation hair removal device 1 configured as described above.

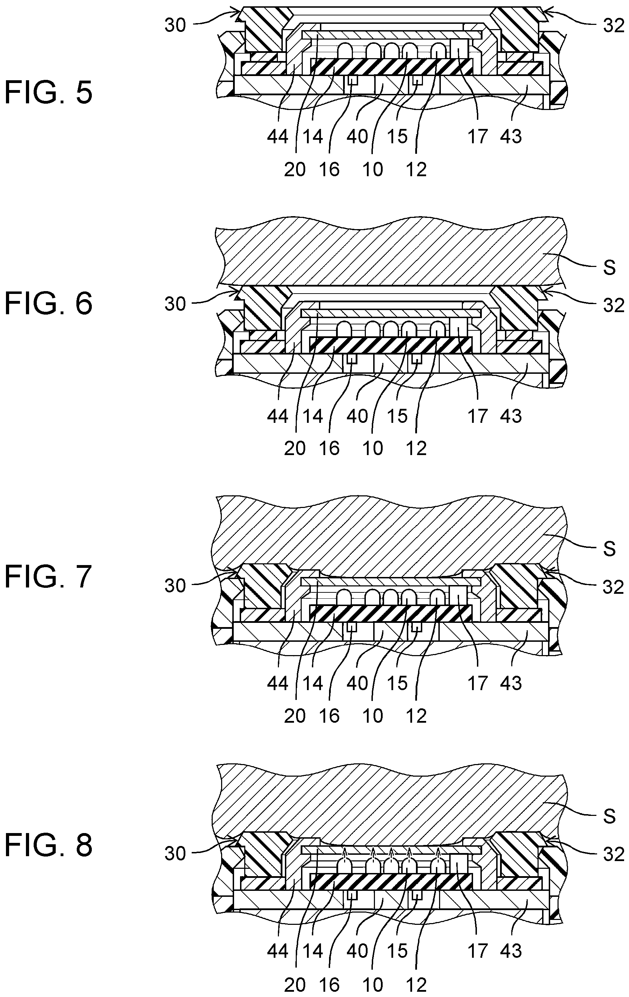

With reference to Figs. 5 to 8, a state in which light is emitted by light irradiation hair removal device 1 will be described. Fig. 5 is a cross-sectional view illustrating one example of a state before use of light irradiation hair removal device 1. Fig. 6 is a cross-sectional view illustrating one example of a state of light irradiation hair removal device 1 before push switch 30 is pressed. Fig. 7 is a cross-sectional view illustrating one example of a state of light irradiation hair removal device 1 after push switch 30 is pressed. Fig. 8 is a cross-sectional view illustrating an example of a state of light irradiation hair removal device 1 while skin S is irradiated with light.

As shown in Fig. 5, push switch 30 is not pressed before use of light irradiation hair removal device 1. Therefore, pressing unit 32 of push switch 30 protrudes toward a direction opposite to first light source 10 and second light source 12 against skin cooling unit 20 from a surface of skin cooling unit 20 in contact with skin S. In this state, light is not emitted from first light source 10 and second light source 12.

As shown in Fig. 6, when light irradiation hair removal device 1 is used, skin S of a user is pressed against light irradiation hair removal device 1. Pressing unit 32 of push switch 30 protrudes from a skin contact surface of skin cooling unit 20. Therefore, skin S of the user first comes into contact with push switch 30, and first light source 10 and second light source 12 are surrounded by skin S and push switch 30.

As shown in Fig. 7, push switch 30 is pressed in contact with skin S. Specifically, in a case where pressing unit 32 of push switch 30 is pressed, the surface pressed by skin S moves toward the direction of first light source 10 and second light source 12 against skin cooling unit 20. As a result, skin S comes into contact with skin cooling unit 20 in a state where first light source 10 and second light source 12 are surrounded by skin S and push switch 30, and skin cooling unit 20 is shielded by skin S. Then, skin S is cooled by coming into contact with skin cooling unit 20.

As shown in Fig. 8, a circuit to which first light source 10 and second light source 12 are connected is closed by push switch 30, and light is emitted from first light source 10 and second light source 12. Since skin cooling unit 20 is shielded by skin S, and first light source 10 and second light source 12 are also surrounded by push switch 30, skin S is irradiated with light emitted from first light source 10 and second light source 12 without leakage. In order to bring skin cooling unit 20 and skin S into contact with each other more reliably, light may be emitted from first light source 10 and second light source 12 after a predetermined time has elapsed since pressing unit 32 of push switch 30 was pressed.

Further, in the present exemplary embodiment, light irradiation hair removal device 1 includes first cooling unit 40 and second cooling unit 41. In the present exemplary embodiment, first light source 10 and second light source 12 include a plurality of LEDs, and the peak wavelength of the LEDs shifts depending on the temperature. For example, when the temperature of the LEDs decreases by about several tens of degrees Celsius, the wavelength of the emitted light decreases by about several tens of nanometers. Therefore, by cooling first light source 10, first cooling unit 40 shifts the wavelength of light emitted from first light source 10 to a wavelength different from the wavelength of light emitted from second light source 12.

By shifting the wavelength of light, a light depth entering skin S changes due to wavelength dependency of light scattering. For example, when the temperature of first light source 10 is lowered and the wavelength of light is shortened, the light depth becomes shallow. On the other hand, since the wavelength of the light of second light source 12 is longer than that of first light source 10, light is less scattered in skin S, and the light depth reaching skin S is deep. Then, the entire melanocytes including melanin of hair follicles are heated when skin S is irradiated with light having different depths. In this way, the wavelengths of light emitted from first light source 10 and second light source 12 can be easily adjusted respectively by independently controlling the temperatures of first light source 10 and second light source 12. Therefore, for light irradiation hair removal device 1 according to the present exemplary embodiment, it is also possible to effectively remove a hair follicle size part where a sufficient hair removal effect cannot be obtained by a device in which only LEDs of different emission wavelengths are arranged.

### [Effect]

As described above, light irradiation hair removal device 1 according to the present exemplary embodiment includes first light source 10, second light source 12, skin cooling unit 20, push switch 30, first cooling unit 40, and second cooling unit 41. First light source 10 emits light having a wavelength from 400 nm to 1200 nm inclusive. Second light source 12 emits light having a wavelength from 400 nm to 1200 nm inclusive. Skin cooling unit 20 faces first light source 10 and second light source 12, transmits light emitted from first light source 10 and second light source 12, and cools skin S in a case where it comes into contact with skin S. Push switch 30 includes pressing unit 32 surrounding a periphery of first light source 10, second light source 12, and skin cooling unit 20. In a case where pressing unit 32 is not pressed, it protrudes toward a direction opposite to first light source 10 and second light source 12 against skin cooling unit 20 from a surface of skin cooling unit 20 in contact with skin S. In a case where pressing unit 32 is pressed, the surface pressed by skin S moves toward the direction of first light source 10 and second light source 12 against skin cooling unit 20. Push switch 30 switches between emission and non-emission of light from first light source 10 and second light source 12. Light is emitted from first light source 10 and second light source 12 during at least a part of time while pressing unit 32 is pressed, and light is not emitted from first light source 10 and second light source 12 while pressing unit 32 is not pressed. First cooling unit 40 cools first light source 10. Second cooling unit 41 cools second light source 12. By cooling first light source 10, first cooling unit 40 shifts the wavelength of light emitted from first light source 10 to a wavelength different from the wavelength of light emitted from second light source 12.

As a result, since first cooling unit 40 cools first light source 10 and second cooling unit 41 cools second light source 12, light irradiation hair removal device 1 can set the temperature of first light source 10 and the temperature of second light source 12 to arbitrary different temperatures. Therefore, since skin S is irradiated with light of different depths due to wavelength dependency of light scattering, light irradiation hair removal device 1 can uniformly irradiate the entire area of melanocytes distributed in hair follicles with light.

Further, light irradiation hair removal device 1 can irradiate skin S with light in a state where first light source 10 and second light source 12 are surrounded by push switch 30 and skin S. Therefore, leakage of light can be suppressed. Further, skin cooling unit 20 can come into contact with skin S to cool skin S at the time of light irradiation. Therefore, inflammation of skin S can be suppressed.

Note that, light irradiation hair removal device 1 may include a near-infrared LED (first light source 10), a push-in irradiation switch (push switch 30), and a skin cooling unit (skin cooling unit 20). The skin cooling unit (skin cooling unit 20) is a transparent material that is cooled on a light beam that is an upper portion of the near-infrared LED (first light source 10) and transmits light of the near-infrared LED (first light source 10). Light irradiation hair removal device 1 has a configuration in which the near-infrared LED (first light source 10) emits light after the skin (skin S) comes into contact with a top surface of the skin cooling unit (skin cooling unit 20) by being pushed into the skin (skin S). Light irradiation hair removal device 1 is characterized in that the wavelength of the near-infrared LED (first light source 10) is changed by arbitrarily controlling the temperature of a part of substrate 14 on which LED elements of the near-infrared LED (first light source 10) are disposed. Even with such light irradiation hair removal device 1, it is possible to uniformly irradiate the entire area of melanocytes distributed in hair follicles with light.

As in the present exemplary embodiment, light irradiation hair removal device 1 may further include first temperature sensor 15 and second temperature sensor 16, which are examples of a temperature sensor according to the present disclosure, and controller 50. First temperature sensor 15 and second temperature sensor 16 may detect the temperature of first light source 10 and the temperature of second light source 12. Controller 50 may control the cooling by first cooling unit 40 so as to cool first light source 10 to a temperature different from the temperature of second light source 12 according to the temperatures of first light source 10 and second light source 12 detected by first temperature sensor 15 and second temperature sensor 16.

As a result, light irradiation hair removal device 1 can more precisely control the temperature of first light source 10. Therefore, light irradiation hair removal device 1 can further precisely control the light depth entering skin S.

As in light irradiation hair removal device 1 according to the present exemplary embodiment, first cooling unit 40 may include a Peltier element, and first temperature sensor 15 and second temperature sensor 16 may include a thermistor.

As a result, light irradiation hair removal device 1 can cool first light source 10 more powerfully and control the temperature of first light source 10 with higher accuracy. Therefore, light irradiation hair removal device 1 can further precisely control the light depth entering skin S.

As in light irradiation hair removal device 1 according to the present exemplary embodiment, first cooling unit 40 may cool first light source 10 so that the temperature of first light source 10 becomes lower than the temperature of second light source 12.

As a result, the wavelength of the light of first light source 10 becomes shorter than the wavelength of the light of second light source 12. Upper portions of hair follicles have more melanocytes than lower portions of hair follicles. Light irradiation hair removal device 1 irradiates the upper portions of hair follicles with light by first light source 10 and irradiates the deep portions of hair follicles with light by second light source 12, so that a light amount according to the melanocyte distribution can be irradiated, and thus the hair removal effect can be further improved.

As in light irradiation hair removal device 1 according to the present exemplary embodiment, first cooling unit 40 may cool first light source 10 so as to be in a predetermined temperature range according to parts of skin S irradiated with light from first light source 10 and second light source 12.

As a result, light irradiation hair removal device 1 can emit light of an optimum wavelength from first light source 10 and second light source 12 according to the parts. Therefore, light irradiation hair removal device 1 can obtain a higher hair removal effect.

### (Other exemplary embodiments)

As described above, the above exemplary embodiment has been described as an example of the technology in the present disclosure. However, the technology of the present disclosure is not limited to them, and is also applicable to exemplary embodiments in which changes, replacements, additions, omissions, and the like are made. Thus, hereinafter, other exemplary embodiments are illustrated as examples.

Light irradiation hair removal device 1 according to the above exemplary embodiment has been described as an example that first light source 10 and second light source 12 include LEDs. However, it is sufficient that first light source 10 and second light source 12 can emit light having a wavelength from 400 nm to 1200 nm inclusive. First light source 10 and second light source 12 are not limited to LEDs, and may be, for example, laser diodes.

Further, skin cooling unit 20 may include an antireflection film for preventing light emitted from first light source 10 and second light source 12 from being reflected. The antireflection film may be provided, for example, on a surface of skin cooling unit 20 facing first light source 10 and second light source 12. By providing such an antireflection film in skin cooling unit 20, reflection of light is suppressed, and skin S can be irradiated with a large amount of light.

Further, light irradiation hair removal device 1 according to the above exemplary embodiment has been described as an example that it cools skin cooling unit 20 using second cooling unit 41. However, in a case where the thermal conductivity of skin cooling unit 20 is high, it is not always necessary to cool skin cooling unit 20 using second cooling unit 41, since the heat dissipation of skin cooling unit 20 is high.

Further, light irradiation hair removal device 1 according to the above exemplary embodiment has been described as an example that second cooling unit 41 is connected to skin cooling unit 20 to cool skin cooling unit 20. However, second cooling unit 41 does not need to be connected to skin cooling unit 20.

Further, as an example, light irradiation hair removal device 1 according to the above exemplary embodiment uses first temperature sensor 15 to measure the temperature of first light source 10. Further, as an example, light irradiation hair removal device 1 according to the above exemplary embodiment uses second temperature sensor 16 to measure the temperature of second light source 12. However, by calculating the thermal conductivity of substrate 14 or the like, it is possible to estimate the temperatures of first light source 10 and second light source 12 with one temperature sensor. Therefore, light irradiation hair removal device 1 does not need to use two temperature sensors, and may include one temperature sensor that measures the temperatures of first light source 10 and second light source 12.

Note that, since the above-described exemplary embodiments are intended to illustrate the technique in the present disclosure, various changes, replacements, additions, omissions, and the like can be made within the scope of claims.

### INDUSTRIAL APPLICABILITY

As described above, the light irradiation hair removal device according to the present disclosure can be applied to, for example, a light irradiation hair removal device for business use and home use.

### REFERENCE MARKS IN THE DRAWINGS

1: light irradiation hair removal device
5: housing
6: first opening portion
7: second opening portion
10: first light source
12: second light source
14: substrate
15: first temperature sensor
16: second temperature sensor
17: third temperature sensor
20: skin cooling unit
30: push switch
31: base
32: pressing unit
40: first cooling unit
41: second cooling unit
42: heat dissipation unit
43: connection unit
44: grip unit
45: heat dissipation fin
46: air blower
50: controller
51: substrate
52: mode selection unit
321: first component
322: second component
S: skin

## Claims

1. A light irradiation hair removal device comprising:
a first light source (10) configured to emit first light having a wavelength from 400 nm to 1200 nm inclusive;
a second light source (12) configured to emit second light having a wavelength from 400 nm to 1200 nm inclusive;
a skin cooling unit (20) that faces the first light source and the second light source, configured to transmit the first light emitted from the first light source and the second light emitted from the second light source, and to cool
a skin when the skin cooling unit comes into contact with the skin;
a push switch (30) that includes a pressing unit (32) surrounding a periphery of the first light source, the second light source, and the skin cooling unit;
wherein
when the pressing unit is not being pressed, the pressing unit protrudes toward a direction opposite to the first light source and the second light source against the skin cooling unit from a surface of the skin cooling unit in contact with the skin;
when the pressing unit is pressed by the skin, a surface of which pressed by the skin moves toward the direction of the first light source and the second light source against the skin cooling unit; and
the push switch is configured to switch between emission and non-emission of the first
light from the first light source and the second light from the second light source such that the first light is emitted from the first light source and the second light is emitted from the second light source during at least a part of time while the pressing unit is pressed, and the first light is not emitted from the first light source and the second light is not emitted from the second light source while the pressing unit is not pressed;
a first cooling unit (40) configured to cool the first light source; and
a second cooling unit (41) configured to cool the second light source, wherein
the first cooling unit is configured to shift
the wavelength of the first light emitted from the first light source to a wavelength different from the wavelength of the second light emitted from the second light source by cooling the first light source.

2. The light irradiation hair removal device according to Claim 1, further comprising:
a temperature sensor (15) configured to detect a first temperature of the first light source and a second temperature of the second light source; and
a controller configured to control a cooling by the first cooling unit so as to cool the first light source to a temperature different from the second temperature of the second light source according to the first temperature of the first light source and the second temperature of the second light source detected by the temperature sensor.

3. The light irradiation hair removal device according to Claim 2, wherein
the first cooling unit includes a Peltier element, and
the temperature sensor includes a thermistor.

4. The light irradiation hair removal device according to any one of Claims 1 to 3, wherein the first cooling unit (40) is configured to cool the first light source, so that the first temperature of the first light source becomes lower than the second temperature of the second light source.

5. The light irradiation hair removal device according to any one of Claims 1 to 4, wherein the first cooling unit (40) is configured to cool the first light source to be in a predetermined temperature range according to parts of the skin irradiated with the first light from the first light source and the second light from the second light source.

## Patentansprüche

1. Vorrichtung für Entfernung von Haaren mittels Lichtbestrahlung, die umfasst:
eine erste Lichtquelle (10), die so ausgeführt ist, dass sie erstes Licht mit einer Wellenlänge von 400 nm bis einschließlich 1.200 nm emittiert;
eine zweite Lichtquelle (12), die so ausgeführt ist, dass sie zweites Licht mit einer Wellenlänge von 400 nm bis einschließlich 1.200 nm emittiert;
eine Einheit (20) zum Kühlen der Haut, die der ersten Lichtquelle und der zweiten Lichtquelle zugewandt und so ausgeführt ist, dass sie das von der ersten Lichtquelle emittierte erste Licht und das von der zweiten Lichtquelle emittierte zweite Licht durchlässt und eine Haut dort kühlt, wo sie in Kontakt mit der Haut kommt;
einen Druckschalter (30), der eine Press-Einheit (32) enthält, die einen Umfang der ersten Lichtquelle, der zweiten Lichtquelle sowie der Einheit zum Kühlen der Haut umschließt; wobei
wenn die Press-Einheit nicht gepresst wird, die Press-Einheit in einer Richtung entgegengesetzt zu der ersten Lichtquelle sowie der zweiten Lichtquelle zu der Einheit zum Kühlen der Haut von einer Fläche der Einheit zum Kühlen der Haut vorsteht, die in Kontakt mit der Haut ist;
wenn die Press-Einheit durch die Haut gepresst wird, sich eine Fläche derselben, die durch die Haut gepresst wird, in der Richtung der ersten Lichtquelle sowie der zweiten Lichtquelle zu der Einheit zum Kühlen der Haut bewegt; und
wobei der Druckschalter so ausgeführt ist, dass er zwischen Emission und Nicht-Emission des ersten Lichtes von der ersten Lichtquelle und des zweiten Lichtes von der zweiten Lichtquelle so umschaltet, dass, während wenigstens eines Teils der Zeit, während die Press-Einheit gepresst wird, das erste Licht von der ersten Lichtquelle emittiert wird und das zweite Licht von der zweiten Lichtquelle emittiert wird, und das erste Licht nicht von der ersten Lichtquelle emittiert wird und das zweite Licht nicht von der zweiten Lichtquelle emittiert wird, während die Press-Einheit nicht gepresst wird;
eine erste Kühl-Einheit (40), die so ausgeführt ist, dass sie die erste Lichtquelle kühlt; sowie
eine zweite Kühl-Einheit (41), die so ausgeführt ist, dass sie die zweite Lichtquelle kühlt, wobei
die erste Kühl-Einheit so ausgeführt ist, dass sie die Wellenlänge des von der ersten Lichtquelle emittierten Lichtes zu einer Wellenlänge verschiebt, die sich von der Wellenlänge des von der zweiten Lichtquelle emittierten zweiten lichtes unterscheidet, indem sie die erste Lichtquelle kühlt.

2. Vorrichtung für Entfemung von Haaren mittels Lichtbestrahlung nach Anspruch 1, die des Weiteren umfasst:
einen Temperatur-Sensor (15), der so ausgeführt ist, dass er eine erste Temperatur der ersten Lichtquelle sowie eine zweite Temperatur der zweiten Lichtquelle erfasst; und
eine Steuerungseinrichtung, die so ausgeführt ist, dass sie eine Kühlung durch die erste Kühl-Einheit entsprechend der ersten Temperatur der ersten Lichtquelle und der zweiten Temperatur der zweiten Lichtquelle, die durch den Temperatursensor erfasst werden, so steuert, dass die erste Lichtquelle auf eine Temperatur gekühlt wird, die sich von der zweiten Temperatur der zweiten Lichtquelle unterscheidet.

3. Vorrichtung für Entfernung von Haaren mittels Lichtbestrahlung nach Anspruch 2, wobei die erste Kühl-Einheit ein Peltier-Element enthält, und
der Temperatursensor einen Thermistor enthält.

4. Vorrichtung für Entfernung von Haaren mittels Lichtbestrahlung nach einem der Ansprüche 1 bis 3, wobei die erste Kühl-Einheit (40) so ausgeführt ist, dass sie die erste Lichtquelle so kühlt, dass die erste Temperatur der ersten Lichtquelle niedriger wird als die zweite Temperatur der zweiten Lichtquelle.

5. Vorrichtung für Entfernung von Haaren mittels Lichtbestrahlung nach einem der Ansprüche 1 bis 4, wobei die erste Kühl-Einheit (40) so ausgeführt ist, dass sie die erste Lichtquelle Teilen der Haut entsprechend, die mit dem ersten Licht von der ersten Lichtquelle und dem zweiten Licht von der zweiten Lichtquelle bestrahlt werden, so kühlt, dass sie sich in einem vorgegebenen Temperaturbereich befindet.

## Revendications

1. Dispositif d'épilation par irradiation lumineuse comprenant :
une première source de lumière (10) configurée pour émettre une première lumière ayant une longueur d'onde comprise entre 400 nm et 1 200 nm inclus ;
une deuxième source de lumière (12) configurée pour émettre une deuxième lumière ayant une longueur d'onde comprise entre 400 nm et 1 200 nm inclus ;
une unité de refroidissement de la peau (20) qui fait face à la première source de lumière et à la deuxième source de lumière, configurée pour transmettre la première lumière émise par la première source de lumière et la deuxième lumière émise par la deuxième source de lumière, et pour refroidir la peau lorsque l'unité de refroidissement de la peau entre en contact avec la peau ;
un interrupteur à poussoir (30) qui inclut une unité de pression (32) entourant une périphérie de la première source de lumière, de la deuxième source de lumière et de l'unité de refroidissement de la peau ;
dans lequel, lorsque l'unité de pression n'est pas pressée, l'unité de pression fait saillie vers une direction opposée à la première source de lumière et à la deuxième source de lumière contre l'unité de refroidissement de la peau à partir d'une surface de l'unité de refroidissement de la peau en contact avec la peau ;
lorsque l'unité de pression est pressée par la peau, une surface pressée par la peau se déplace vers la direction de la première source de lumière et de la deuxième source de lumière par rapport à l'unité de refroidissement de la peau ; et
l'interrupteur à poussoir est configuré pour commuter entre l'émission et la non-émission de la première lumière provenant de la première source de lumière et de la deuxième lumière provenant de la deuxième source de lumière de sorte que la première lumière est émise par la première source de lumière et la deuxième lumière est émise par la deuxième source de lumière pendant au moins une partie du temps lorsque l'unité de pression est pressée, et que la première lumière n'est pas émise par la première source de lumière et la deuxième lumière n'est pas émise par la deuxième source de lumière lorsque l'unité de pression n'est pas pressée ;
une première unité de refroidissement (40) configurée pour refroidir la première source de lumière ; et
une deuxième unité de refroidissement (41) configurée pour refroidir la deuxième source de lumière,
dans lequel la première unité de refroidissement est configurée pour déplacer la longueur d'onde de la première lumière émise par la première source de lumière à une longueur d'onde différente de la longueur d'onde de la deuxième lumière émise par la deuxième source de lumière en refroidissant la première source de lumière.

2. Dispositif d'épilation par irradiation lumineuse selon la revendication 1, comprenant en outre :
un capteur de température (15) configuré pour détecter une première température de la première source de lumière et une deuxième température de la deuxième source de lumière ; et
un contrôleur configuré pour commander un refroidissement par la première unité de refroidissement de manière à refroidir la première source de lumière à une température différente de la deuxième température de la deuxième source de lumière selon la première température de la première source de lumière et la deuxième température de la deuxième source de lumière détectées par le capteur de température.

3. Dispositif d'épilation par irradiation lumineuse selon la revendication 2, dans lequel
la première unité de refroidissement inclut un élément Peltier, et
le capteur de température inclut une thermistance.

4. Dispositif d'épilation par irradiation lumineuse selon l'une quelconque des revendications 1 à 3, dans lequel la première unité de refroidissement (40) est configurée pour refroidir !la première source de lumière, de sorte que la première température de la première source de lumière devienne inférieure à la deuxième température de la deuxième source de lumière.

5. Dispositif d'épilation par irradiation lumineuse selon l'une quelconque des revendications 1 à 4, dans lequel la première unité de refroidissement (40) est configurée pour refroidir la première source de lumière afin qu'elle se trouve dans une plage de températures prédéterminée selon les parties de la peau irradiées par la première lumière provenant de la première source de lumière et par la deuxième lumière provenant de la deuxième source de lumière.
